Europäisches Patentamt

European Patent Office

Office européen des brevets

(11) Numéro de publication: **0 093 027**
**A1**

## (12) DEMANDE DE BREVET EUROPEEN

(21) Numéro de dépôt: **83400596.9**

(22) Date de dépôt: **23.03.83**

(51) Int. Cl.³: **C 04 B 35/18**
**C 12 N 11/14, C 12 N 5/00**

(30) Priorité: 27.04.82 FR 8207205
21.09.82 FR 8215845

(43) Date de publication de la demande:
02.11.83 Bulletin 83/44

(84) Etats contractants désignés:
CH DE GB LI NL SE

(71) Demandeur: ARGILES & MINERAUX AGS-BMP
Clerac
F-17270 Montguyon(FR)

(72) Inventeur: Geirnaert, Gilles
17 rue de l'Arquebuse
F-17420 Saint-Palais-sur-Mer(FR)

(74) Mandataire: Cuer, André
CABINET CUER 30, rue de Léningrad
F-75008 Paris(FR)

(54) **Support de fixation de micro-organismes.**

(57) Support céramique pour l'immobilisation d'enzymes et/ou microorganismes tels que champignons, bactéries, levures, virus . . . etc.

Le support est constitué par un silico-aluminate renfermant 5 à 25 % de magnésie et de granulométrie 5 à 300 microns. Avantageusement, il renferme plus de 50 % en poids de cordiérite synthétique.

Application à l'immobilisation de catalyseurs biologiques et de cellues d'hydridomes pour la production d'anticorps monoclonaux.

EP 0 093 027 A1

La présente invention a trait au domaine des matériaux anorganiques utilisés comme supports insolubles pour l'immobilisation d'enzymes et/ou de micro-organismes tels que champignons, bactéries, levures, virus ou tout autre matière vivante mono ou polycellulaire. Elle concerne plus spécialement un nouveau support de type céramique, contenant au moins 50 % de cordiérite, de caractéristiques bien particulières.

Il est connu de longue date de préparer et utiliser des supports, notamment en matériaux minéraux plus ou moins poreux, destinés à fixer, par adsorption ou autre phénomène, des populations microbiennes telles que bactéries, levures, enzymes ou autres et de constituer ainsi des catalyseurs biologiques utilisables pour de multiples applications dans les domaines industriels et agricoles les plus divers.

On a ainsi préconisé, entre autres supports, des matériaux vitreux frittés, des matières cristallines telles que spinelle-zircone ou cordiérite. Il a été bien précisé, dans ce cas, que ces produits devaient avoir, avec un taux d'au moins 70 %, des diamètres moyens de pores bien délimités tels que notamment 0,8 à 220 microns pour une population de bactéries et 1 à 140 microns pour des cellules de levure ; en outre, la granulométrie annoncée pour les supports était de 0,7 à 1 mm (brevet français n° 78.26415 publié sous n° 2403384 ; brevet américain n° 4 149 937).

Il a maintenant été trouvé que, dans le cas tout au moins de certains matériaux anorganiques, la quantité de biomasse fixée par unité de surface du matériau ainsi que la stabilisation dans le temps du biocatalyseur obtenu n'étaient pas nécessairement fonction de la porosité du produit ni de la nécessité d'avoir à sa disposition un diamètre moyen de pores au moins égal et de préférence plusieurs fois supérieur à un diamètre du micro-organisme à fixer. L'efficacité du catalyseur peut en effet se trouver profondément affectée par des phénomènes de restriction d'accessibilité, mécaniques ou physiques, pour les organismes ; par exemple, dans les supports à grande porosité, les enzymes qui tapissent la paroi des pores peuvent montrer une activité très réduite du fait notamment de la constitution d'une couche limite de diffusion à la surface du support solide, cette couche constituant une barrière qui ralentit les réactions provoquées par la biomasse.

Le but de l'invention est de proposer un support qui, sans être adapté aux exigences de porosité précitées, soit apte à fournir non seulement une activité importante dans un milieu réactionnel mais à manifester une grande stabilité dans le temps, notamment en donnant des taux de conversion -dans l'utilisation comme biocatalyseur- qui soient constant,pendant une longue durée, par exemple de un ou plusieurs mois.

Le nouveau support selon l'invention est essentiellement constitué par un silico-aluminate renfermant des teneurs bien déter - minées en magnésie, à savoir entre 5 et 25 % (en poids), et dont la granulométrie moyenne est comprise entre 5 et 300 microns, avec un intervalle tel que le diamètre des plus gros grains soit au maximum deux fois celui des plus petits, et avantageusement inférieur à une fois et demie.

Avantageusement, un tel support est, au point de vue cristallographique, du type cordiérite synthétique dans laquelle la teneur en fer, exprimée en $Fe_2O_3$ , est inférieure à 2 %. Bien qu'il puisse être obtenu par d'autres méthodes ou variantes, ce matériau est avantageusement préparé par malaxage d'argile kaolinique avec du talc ou du carbonate de magnésium puis calcination vers 1000 à 1400°C et broyage fin du matériau céramique obtenu pour obtenir la fourchette de granulométrie désirée. Par exemple, si l'on utilise du talc, la quantité sera avantageusement voisine de 30 % en poids (pour 70 % d'argile) alors qu'elle sera de l'ordre de 20 % pour le carbonate de magnésium. On peut aussi accélérer la cuisson et diminuer le palier de température de cette dernière en incorporant à la pâte de cordiérisation un petit pourcentage, par exemple 5 à 10 %, de cordiérite synthétique finement broyée par exemple de type ARTAL 23 (marque déposée) qui agit comme germe de cristallisation.

Parmi les nombreux cas d'application des supports selon l'invention, on décrira ci-dessous en détail deux exemples non limitatifs illustrant l'un la fixation de bactéries pour la production de protéines et l'autre, la formation de macro-molécules, par exemple des anticorps, secrétées par des organismes vivants.

Exemple n° 1

Cet exemple concerne la réalisation d'un bioréacteur en continu pour la production de tryptophane par immobilisation de cellules d'Echerichia coli sur un support de l'invention, en utilisant de l'indole comme substrat. On a également effectué des comparaisons avec d'autres supports ne satisfaisant pas aux paramètres critiques de l'invention.

a) Supports mis en oeuvre

Le support de l'invention, dénommé ci-après A, a été préparé par mélange de 70 parties d'argile kaolinique ($KR_2$) et 30 parties de talc (2 C) puis cuisson à température de 1350°C et broyage-criblage pour ne retenir que les particules et grains fins présentant une fourchette de granulométrie entièrement comprise entre 100 et 200 microns. Sa composition chimique était la suivante : $SiO_2$:53, 7 ; $Al_2O_3$: 30, 8 ; $Fe_2O_3$:1, 6 ; $TiO_2$:1, 1 ; $K_2O + Na_2O$:0, 6 ; $Mg_0$:9, 6. Quant à la structure minéralogique, elle correspondait sensiblement à 80 % de cordiérite, 8 % de mullite et 12 % de verre.

A titre de comparaison, on a également effectué les mêmes expériences avec deux supports de type silico-aluminates de compositions assez voisines de celles du produit A mais contenant très peu de magnésie, à savoir :

Support L    $SiO_2$:54, 3 ; $Al_2O_3$: 41, 3 ; $FeO_3$: 1, 9 ; $TiO_2$:1, 4 ; $K_2O + Na_2O$:0, 7 ; $CaO + MgO$:0, 4.

Support X    $SiO_2$:53, 6 ; $Al_2O_3$:44, 2 ; $Fe_2O_3$:0, 9 ; $TiO_2$:0, 8 ; $K_2O + Na_2O$:0, 3 ; $CaO + MgO$:0, 2.

Au plan cristallographique, ces deux supports contenaient des quantités sensiblement équivalentes de mullite, cristoballite et verre. En outre, environ 70 % des pores de ces compositions céramiques avaient un diamètre moyen compris entre 1 et 200 microns, pour se rapprocher des conditions du brevet français précité (publication n° 2 403 384).

b) Production de tryptophane

On est parti de bactéries d'Echerichia coli, qui présentaient une activité tryptophanase élevée avec une concentration de 0, 3 g/l de tryptophane comme inducteur dans le milieu de fermentation. Les bactéries ont été utilisées en suspension pour tester le système enzymatique de conversion de l'indole en tryptophane dans un milieu de composition suivante :

indole $10^{-2}$ mole ; pyruvate de sodium : 0, 5 mole ; acétate d'ammonium : 0, 6 mole ; phosphate de pyridoxal : $10^{-3}$ mole ; éthanol : 10 %. Le pH était de 9 et la température de 37°C. On a testé les différents supports dans un réacteur à lit fixe en fixant par adsorption les bactéries pendant plusieurs heures par circulation de la suspension bactérienne à débit de 30 ml/h dans un tampon de phosphate 0, 1 M (pH 7). On effectuait un lavage avec ce tampon phosphate pour éliminer l'excès de bactéries jusqu'à l'obtention d'une diffraction optique nulle en sortie.

La colonne étant prête, on a introduit le substrat dans les conditions précitées puis on a suivi la cinématique enzymatique par mesure, en fonction du temps de la concentration du tryptophane et de l'indole. On a tracé alors les courbes donnant en abscisse les taux de production de tryptophane (en % en poids) par rapport au temps.

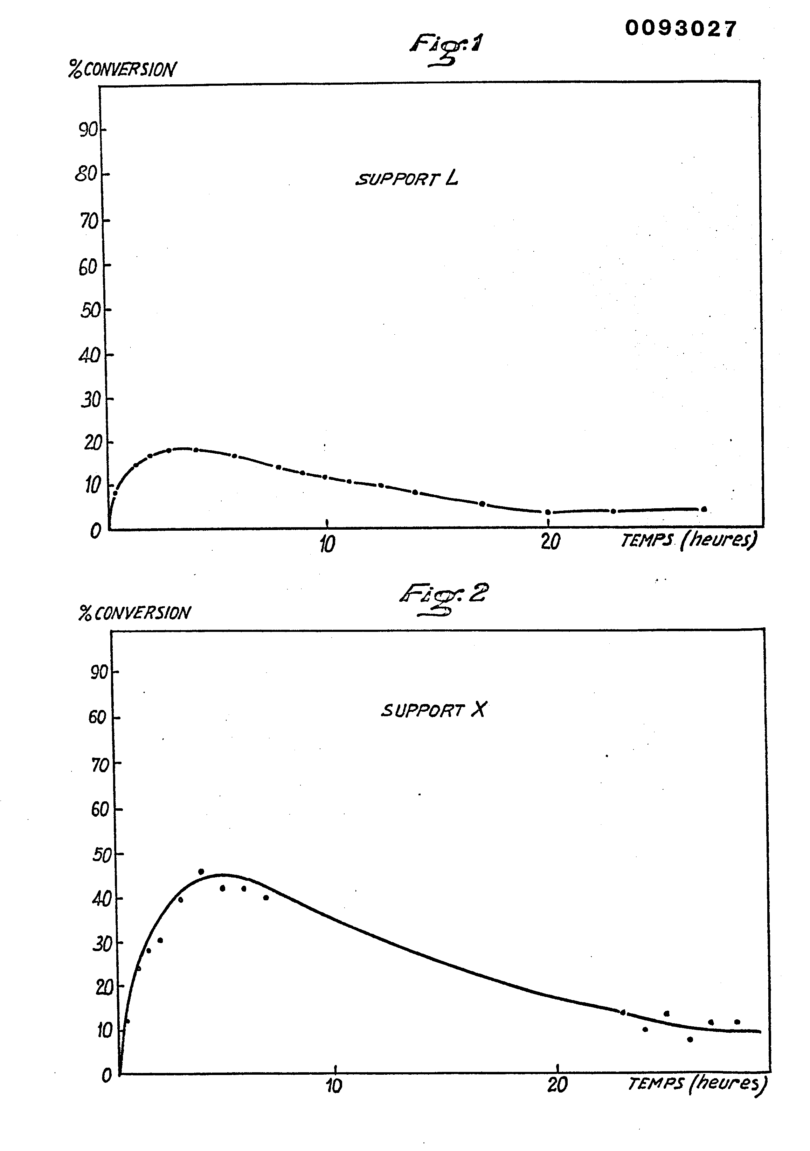

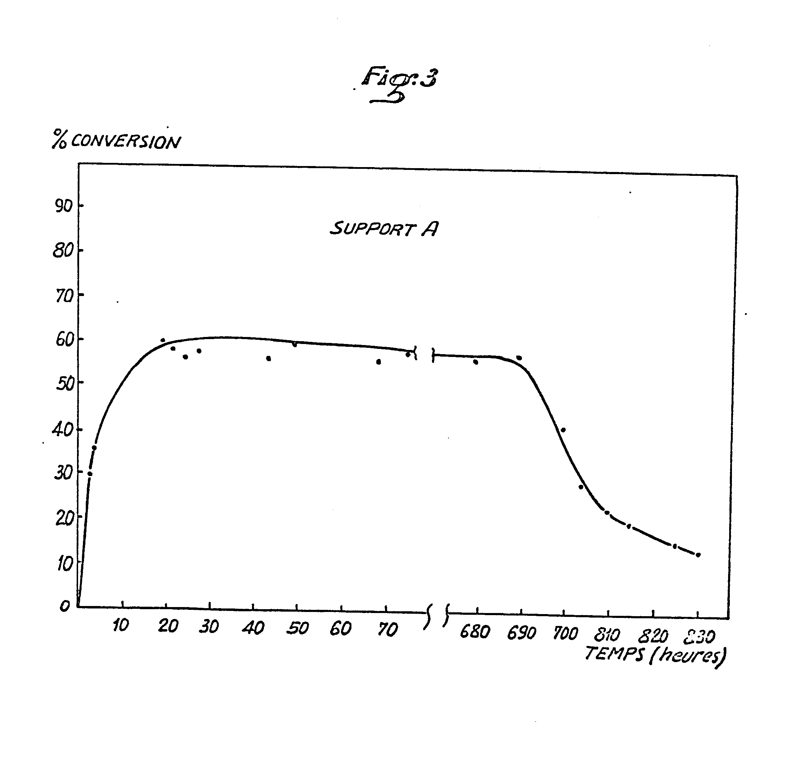

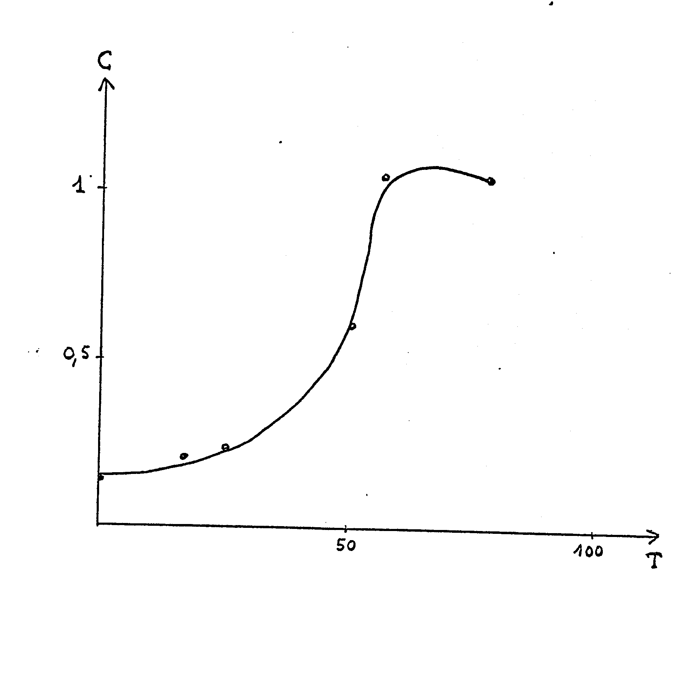

Comme on peut le voir sur les dessins des figures 1 à 3 annexées, le support X donne un rendement de bioconversion de 43 % environ, mais celui-ci ne dure que 3 à 4 heures. Dans ce cas du support L, le rendement maximum est très faible, de l'ordre de 20 %, pour un état stationnaire assez court de 2 à 5 heures. Dans la mise en oeuvre, au contraire, du support A selon l'invention, on a obtenu des résultats très intéressants : en effet, le taux de conversion est resté pratiquement constant, à environ 60 % pendant une durée de 1 mois sans avoir besoin de recharger la colonne en bactéries et sans que l'on constate la présence d'indole à la sortie du réacteur.

Ainsi, les supports selon l'invention permettent de réaliser l'immobilisation de bactéries entières et d'obtenir des catalyseurs biologiques autorisant la fabrication de divers produits dans des procédés continus, avec des rendements compatibles avec les contraintes économiques de l'industrie.

Exemple n° 2

Cet exemple illustre un autre cas important d'utilisation des supports selon l'invention, notamment pour l'immobilisation d'organismes monocellulaires comme par exemple des trypanosomes ou des leishmanias ou, mieux encore, pour l'immobilisation d'hybridomes en vue de la production d'anticorps monoclonaux qui, comme on le sait, servent notamment à l'élaboration de sérums.

On connaît la fragilité et les contraintes de diffusion des cellules d'hybridomes qui résultent de la fusion de lymphocytes et de cellules myélomateuses et permettent la préparation d'anticorps monoclonaux très précieux en immunothérapie humaine (voir par exemple la revue "Pour la Science" novembre 1981 p. 38-39). La culture de ces cellules peut se faire de diverses façons pour les phases de croissance puis de production d'anticorps, par exemple par injection intrapéritonéale d'hybridomes dans la cavité abdominale d'une souris ou par culture en discontinu sur des microbilles d'un support en lit fluidisé.

Il a maintenant été trouvé que les procédés de fractionnement et de purification de molécules biologiques pouvaient être notablement simplifiés grâce à la mise en oeuvre, comme supports de cellules, des matériaux granulaires anorganiques selon l'invention. En effet, grâce aux travaux effectués en collaboration avec l'Institut de Technologie des Surfaces Actives de Compiègne (France), il a pu être mis au point une technique de fixation d'hybridomes et de fabrication d'anticorps monoclonaux par l'utilisation de colonnes en lit fixe de support selon l'invention et fonctionnant en continu. Cette technique présente de nombreux avantages tels que, en particulier : la séparation du milieu de culture, plus simple que dans les cas classiques ; la plus grande facilité de maintenir la stérilité du milieu ; et bien entendu la production en continu d'anticorps monoclonaux.

Dans l'exemple de réalisation ci-après, on a utilisé

6

comme support d'immobilisation de cellules, dans un réacteur cylindrique à lit fixe, le produit dénommé A dont les caractéristiques ont été fournies ci-avant.

On a utilisé comme matériel un réacteur cylindrique thermostaté de 20 cm de hauteur et de 1 cm de diamètre, rempli du support A (20 grammes) et connecté, par l'intermédiaire d'une pompe péristaltique, à un échangeur de gaz ou colonne d'aération apte à fournir au milieu de culture un gaz contenant approximativement : 74 % d'azote, 20 % d'oxygène et 6 % de gaz carbonique.

Avant opération, le support A a été lavé à l'Hcl puis séché et pasteurisé à 80-90°C dans la colonne pendant 2 heures, avec circulation à cette température d'une solution tampon de phosphate alcalin donnant un pH de 7,2 à 7,4.

Les hybridomes étaient obtenus par fusion de lymphocytes de souris type BALB/C avec des cellules de myélome type $SP_2$ et le concentrat de matière active était introduit dans 150 ml d'un milieu de culture connu RPMI 1640 contenant 10 % de sérum fétal de veau.

Le milieu actif était alors introduit dans le réacteur avec un débit d'environ 1 ml/minute correspondant à une vitesse linéaire de 1,3 cm/minute. Les mesures d'anticorps obtenus (immunoglobulines G) étaient faites sur plaques de nylon hydrolysé et acétylé (avec comparaison d'étalons sur différentes plaques) puis comptage dans une chambre de THOMA et mesure d'ATP (adénosine triphosphate) par luminescence.

Le tableau 1 ci-dessous montre le métabolisme énergique du milieu de culture au cours du processus d'immobilisation des cellules sur le support utilisé :

Tableau 1

|  | A l'entrée du milieu de culture | A la sortie du milieu de culture |
|---|---|---|
| Concentration en cellules $10^5$/ml | 4,6 | 0 |
| ATP (PG/ml) | $8,6 \times 10^3$ | 0 |

L'absence de cellules et d'ATP dans le milieu sortant du réacteur montre clairement que la totalité de la biomasse a été mobilisée dans ce dernier, par un seul passage, ce qui a été confirmé par utilisation du microscope électronique.

Par ailleurs, l'activité des cellules d'hydridomes immobilisées sur le support de l'invention a montré une secrétion continue d'anticorps monoclonaux selon une phase stationnaire comme cela ressort de la courbe (1) de la figure 4 annexée où l'abscisse T correspond au temps en heures alors que l'ordonnée représente la concentration C des immunoglobulines (exprimées en microgrammes par ml).

Ces essais ont donc montré la possibilité d'utilisation, en phase continue, d'un réacteur à lit fixe de support selon l'invention, pour la production d'anticorps et, partant de là, de diverses macro-molécules secrétées par des organismes vivants.

0093027

REVENDICATIONS

1. Support céramique pour la fixation de micro-organismes, caractérisé en ce qu'il est constitué par un silicoaluminate renfermant des teneurs de 5 à 25 % en magnésie et dont la granulométrie moyenne est comprise entre 5 et 300 microns, avec un intervalle tel que le diamètre des plus gros grains soit au maximum deux fois celui des plus petits, et avantageusement inférieur à une fois et demie.

2. Support céramique selon la revendication 1, caractérisé en ce qu'il est obtenu par calcination entre 1000 - 1400°C d'un mélange d'argile kaolinique et de talc ou carbonate de magnésium puis broyage et classification à la répartition granulométrique désirée du matériau final, contenant plus de 50 % de cordiérite.

3. Support selon la revendication 2, caractérisé en ce que l'on mélange environ 70 % (en poids) d'argile kaolinique et 30 % de talc ou encore 80 % d'argile kaolinique et 20 % de carbonate de magnésium.

4. Support selon l'une quelconque des revendications 1 à 3, caractérisé en ce qu'il renferme : 53,7 % de $SiO_2$ ; 30,8 % d'$Al_2O_3$ ; 1,6 % de $FeO_3$ ; 1,1 % de $TiO_2$ ; 0,6 % de $Na_2O + K_2O$ et 9,6 % de $MgO$ ; la structure minéralogique correspondant sensiblement à 80 % de cordiérite, 8 % de mullite et 12 % de verre ; la granulométrie étant entièrement comprise entre 100 et 200 microns.

5. Application du support selon l'invention à l'immobilisation de catalyseurs biologiques pour la production en continu de diverses substances en bioréacteur, ainsi que pour l'immobilisation d'hydridomes en vue de la production d'anticorps monoclonaux.

Fig.1

0093027

% CONVERSION

SUPPORT L

TEMPS (heures)

Fig.2

% CONVERSION

SUPPORT X

TEMPS (heures)

Fig:3

% CONVERSION

SUPPORT A

TEMPS (heures)

0093027

Office européen
des brevets

**RAPPORT DE RECHERCHE EUROPEENNE**

**0093027**
Numéro de la demande

EP  83 40 0596

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| Y | FR-A-2 272 965  (CORNING GLASS WORKS) <br> * Page 1, ligne 1 - page 5, ligne 40 * | 1-4 | C 04 B  35/18 <br> C 12 N  11/14 <br> C 12 N  5/00 |
| | --- | | |
| Y | FR-A-2 289 467  (GENERAL MOTORS CORPORATION) <br> * Page 1, ligne 1 - page 3, ligne 17 * | 1-4 | |
| | --- | | |
| Y | FR-A-2 347 321  (NGK INSULATORS) <br> * Page 1, ligne 1 - page 12, ligne 5 * | 1-4 | |
| | --- | | |
| Y | FR-A-2 376 088  (NGK INSULATORS) <br> * Page 1, ligne 1- page 8, ligne 26 * | 1-4 | **DOMAINES TECHNIQUES RECHERCHES (Int. Cl. ³)** |
| | --- | | |
| Y | US-A-3 967 971  (RICHARD A. EPPLER) <br> * Colonne 1, ligne 5 - colonne 7, ligne 23 * | 1-4 | C 04 B <br> C 12 N |
| | --- | | |
| Y | US-A-4 235 855  (JOSEPH J. CLEVELAND) <br> * Colonne 1, ligne 5 - colonne 4, ligne 3 * | 1-4 | |
| | ---          -/- | | |

Le présent rapport de recherche a été établi pour toutes les revendications

| Lieu de la recherche <br> LA HAYE | Date d'achèvement de la recherche <br> 22-06-1983 | Examinateur <br> REMPP G.L.E. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82

## DOCUMENTS CONSIDERES COMME PERTINENTS

Page  2

| Catégorie | Citation du document avec indication, en cas de besoin, des parties pertinentes | Revendication concernée | CLASSEMENT DE LA DEMANDE (Int. Cl. 3) |
|---|---|---|---|
| Y | EP-A-0 003 923  (RHONE-POULENC INDUSTRIES) * Page 1, ligne 5 - page 4, ligne 37 * | 5 | |
| Y,D | FR-A-2 403 384  (CORNING GLASS WORKS) * Page 1, ligne 1 - page 14, ligne 5 * | 5 | |
| A | FR-A-2 451 942  (K.K. HAYASHIBARA SEIBUTSU KAGAKO KENKYUJO) | | |
| A | FR-A-2 133 370  (PIERRE MONSAN et al.) | | |
| A | FR-A-2 393 810  (KALI-CHEMIE AKTIENGESELLSCHAFT) | | DOMAINES TECHNIQUES RECHERCHES (Int. Cl. 3) |

Le présent rapport de recherche a été établi pour toutes les revendications

| Lieu de la recherche LA HAYE | Date d'achèvement de la recherche 22-06-1983 | Examinateur REMPP G.L.E. |
|---|---|---|